(19) Europäisches Patentamt
European Patent Office
Office européen des brevets

(11) **EP 4 483 798 A1**

(12) **EUROPEAN PATENT APPLICATION**
published in accordance with Art. 153(4) EPC

(43) Date of publication:
01.01.2025 Bulletin 2025/01

(21) Application number: 23760113.3

(22) Date of filing: 24.02.2023

(51) International Patent Classification (IPC):
*A61B 5/0537* (2021.01)

(52) Cooperative Patent Classification (CPC):
A61B 5/0531; A61B 5/01; A61B 5/0533;
A61B 5/0537

(86) International application number:
PCT/JP2023/006774

(87) International publication number:
WO 2023/163115 (31.08.2023 Gazette 2023/35)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 24.02.2022 JP 2022026849

(71) Applicant: Tanita Corporation
Tokyo 174-8630 (JP)

(72) Inventors:
• YAMADA, Yuki
Tokyo 174-8630 (JP)
• KODAMA, Miyuki
Tokyo 174-8630 (JP)
• SAKAI, Yoshio
Tokyo 174-8630 (JP)

(74) Representative: Eisenführ Speiser
Patentanwälte Rechtsanwälte PartGmbB
Postfach 10 60 78
28060 Bremen (DE)

(54) **ANALYSIS DEVICE, ANALYSIS METHOD, ANALYSIS PROGRAM, AND COMPUTER-READABLE NON-TRANSITORY STORAGE MEDIUM**

(57) An analysis device (10) is made to be wearable on a person to be measured and is shaped like, for example, a wristband. The analysis device (10) calculates the BI of a person to be measured based on a measurement result obtained by using a plurality of electrode units (20) being in contact with the person to be measured, and determines a component ingested by the person to be measured based on reference information indicating a temporal change in BI according to a component ingested by a human and on a temporal change in the calculated BI.

FIG. 1

EP 4 483 798 A1

**Description**

CROSS-REFERENCE TO RELATED APPLICATION

This application claims the benefit of Japanese Patent Application No. 2022-026849 filed on February 24, 2022 in Japan, the contents of which are incorporated herein by reference.

TECHNICAL FIELD

The present disclosure relates to an analysis device, an analysis method, and an analysis program.

BACKGROUND ART

One way to know nutrients, minerals, or water ingested by a person during a meal, hydration, and the like is by determining components included in the ingesta from diet records and diet image records and calculating nutrition.
The conventional method mentioned above would require time and effort to create diet records or image records, and might yield results that do not align with reality since the ingesta are not actually measured. Even if the contents of a meal are recorded accurately in detail, the substances in the record may not be nutrients actually included in the meal. Besides, whether the nutrients included in the meal have been actually absorbed by the human body or not cannot be known.
Meanwhile, Patent document 1 (Japanese Patent Laid-Open Application No. 2000-23936), for example, describes a health management guidance device that converts a measured value of the bioimpedance of a person to be measured to a circulatory condition index indicating the circulatory condition of the person to be measured. The device described in Patent document 1 converts an impedance value to a circulatory condition index based on the pattern of the diurnal variation in the impedance.

SUMMARY OF THE INVENTION

Problems to be solved by the invention

As in Patent document 1, the physical condition of a person to be measured can be identified more directly by using the impedance of the person to be measured. What is determined in Patent document 1, however, is the circulatory condition of a person to be measured, and is not a component ingested by a person to be measured.
A purpose of the disclosure made in view of the above is to provide an analysis device, an analysis method, and an analysis program that are able to more accurately identify components ingested by a person to be measured.

Means for solving the problems

An analysis device of an aspect of the disclosure comprises: calculation means for calculating electric resistance of a person to be measured based on a measurement result obtained by using a plurality of electrode units being in contact with the person to be measured; and determination means for determining a component ingested by the person to be measured based on reference information indicating a temporal change in the electric resistance according to a component ingested by a human and on a temporal change in the electric resistance calculated by the calculation means.
With this configuration, a component ingested by a person to be measured is determined by using a phenomenon of the electric resistance of a person to be measured changing in accordance with a component ingested by the person to be measured. This configuration thus allows an ingested component to be identified more accurately without a person to be measured recording the contents of a meal.
An analysis device of an aspect of the disclosure comprises: calculation means for calculating electric resistance of a person to be measured based on a measurement result obtained by using a plurality of electrode units being in contact with the person to be measured; and determination means for determining whether the person to be measured has ingested a specified component or not based on reference information indicating a temporal change in the electric resistance according to a component ingested by a human and on a temporal change in the electric resistance calculated by the calculation means. This configuration allows a person to be measured to recognize whether the person has ingested a specified component or not.
In the analysis device described above, the reference information may be first reference information that indicates a temporal change in the electric resistance for each component resulting from a human ingesting the component, which is protein, fat, or carbohydrate, and the determination means may determine the component ingested by the person to be measured based on a temporal change in the electric resistance calculated by the calculation means and on a temporal change for each component indicated by the first reference information. This configuration allows for identifying which of protein, fat, or carbohydrate a person to be measured has ingested as a nutrient.

In the analysis device described above, the first reference information may include a relation between an amount of the ingested component, which is protein, fat, or carbohydrate, and time until the electric resistance returns to a level before ingestion of the component, and the determination means may determine an amount of the component ingested by the person to be measured based on time until the electric resistance calculated by the calculation means returns to a level before ingestion of the component and on the first reference information. This configuration allows for qualitatively determining the amount of nutrients ingested by a person to be measured.

The analysis device described above may have correction means for correcting an amount of the component determined by the determination means based on variations over one day or more in body composition of the person to be measured. This configuration allows for more accurately identifying a component ingested by a person to be measured.

In the analysis device described above, the first reference information may be generated by acquiring a temporal change in the electric resistance within a predetermined period of time after the person to be measured ingests protein, fat, or carbohydrate. This configuration allows for more accurately identifying a component ingested by a person to be measured through generating the first reference information according to the person to be measured.

The analysis device described above may have body temperature acquisition means for acquiring body temperature of the person to be measured, where the first reference information may include a temporal change in the body temperature for each component resulting from a human ingesting protein, fat, or carbohydrate, and where the determination means may determine the component ingested by the person to be measured based on a combination of the body temperature acquired by the body temperature acquisition means and the electric resistance calculated by the calculation means and on the first reference information. This configuration allows for more accurately identifying a component ingested by a person to be measured.

The analysis device described above may have body temperature acquisition means for acquiring body temperature of the person to be measured, where the determination means may determine a physical condition of the person to be measured based on a relation between a temporal change in the body temperature acquired by the body temperature acquisition means and a temporal change in the electric resistance calculated by the calculation means and on the reference information. This configuration allows for easily identifying a change in the physical condition of a person to be measured.

In the analysis device described above, the reference information may be second reference information indicating that the body temperature increases before the electric resistance increases and that ingestion of water causes the body temperature to decrease along with the electric resistance. This configuration allows for identifying a person to be measured as being dehydrated.

In the analysis device described above, the reference information may be third reference information indicating that there is no temporal change in the body temperature while the electric resistance increases and then decreases. This configuration allows for identifying a person to be measured as having edema due to excessive salt intake.

The analysis device described above may have advice derivation means for deriving advice to maintain health for the person to be measured according to a determination result obtained by the determination means. This configuration allows for presenting an appropriate advice according to a component ingested by a person to be measured.

In the analysis device described above, the electrode units may be made to be wearable on the person to be measured. This configuration allows for identifying a component ingested by a person to be measured regardless of where the person eats.

An analysis method of an aspect of the disclosure has: a first process for calculating electric resistance of a person to be measured based on a measurement result obtained by using a plurality of electrode units being in contact with the person to be measured; and a second process for determining a component ingested by the person to be measured based on reference information indicating a temporal change in the electric resistance according to a component ingested by a human and on a temporal change in the electric resistance calculated by the first process.

An analysis program of an aspect of the disclosure causes a computer to function as: calculation means for calculating electric resistance of a person to be measured based on a measurement result obtained by using a plurality of electrode units being in contact with the person to be measured; and determination means for determining a component ingested by the person to be measured based on reference information indicating a temporal change in the electric resistance according to a component ingested by a human and on a temporal change in the electric resistance calculated by the calculation means.

An analysis method of an aspect of the disclosure has: a first process for calculating electric resistance of a person to be measured based on a measurement result obtained by using a plurality of electrode units being in contact with the person to be measured; and a second process for determining whether the person to be measured has ingested a specified component or not based on reference information indicating a temporal change in the electric resistance according to a component ingested by a human and on a temporal change in the electric resistance calculated by the first process.

An analysis program of an aspect of the disclosure causes a computer to function as: calculation means for calculating electric resistance of a person to be measured based on a measurement result obtained by using a plurality of electrode units being in contact with the person to be measured; and determination means for determining whether the person to be measured has ingested a specified component or not based on reference information indicating a temporal change in the electric resistance according to a component ingested by a human and on a temporal change in the electric resistance calculated by the calculation means.

Advantage of the invention

The disclosure allows for more accurately identifying a component ingested by a person to be measured.

BRIEF DESCRIPTION OF THE DRAWINGS

Figure 1 is a schematic configuration diagram of an analysis device of a first embodiment;
Figure 2 is a function block diagram of the analysis device of the first embodiment;
Figure 3 shows a relation between BI and body temperature;
Figure 4 shows temporal changes of BI for nutrients ingested by a human, where (A) is for fat, (B) is for carbohydrate, and (C) is for protein;
Figure 5 shows temporal changes of BI and body temperature according to physical conditions of a human, where (A) is for a dehydrated human, and (B) is for a human with excessive salt;
Figure 6 is a flowchart showing a flow of an analysis process of the first embodiment;
Figure 7 is a schematic configuration diagram of an analysis system of a second embodiment;
Figure 8 is a function block diagram relating to analysis functions of the second embodiment;
Figure 9 is a schematic diagram relating to an ingested component correction function of the second embodiment;
Figure 10 is a function block diagram relating to analysis functions of a third embodiment; and
Figure 11 is a schematic configuration diagram of an analysis device of another embodiment.

MODES OF EMBODYING THE INVENTION

Embodiments of the disclosure will now be described with reference to the drawings. The embodiments described below are merely illustrative of ways to implement the disclosure, and do not limit the disclosure to the specific configurations described below. When the disclosure is to be implemented, any specific configuration may be appropriately adopted according to the mode of implementation.

(First embodiment)

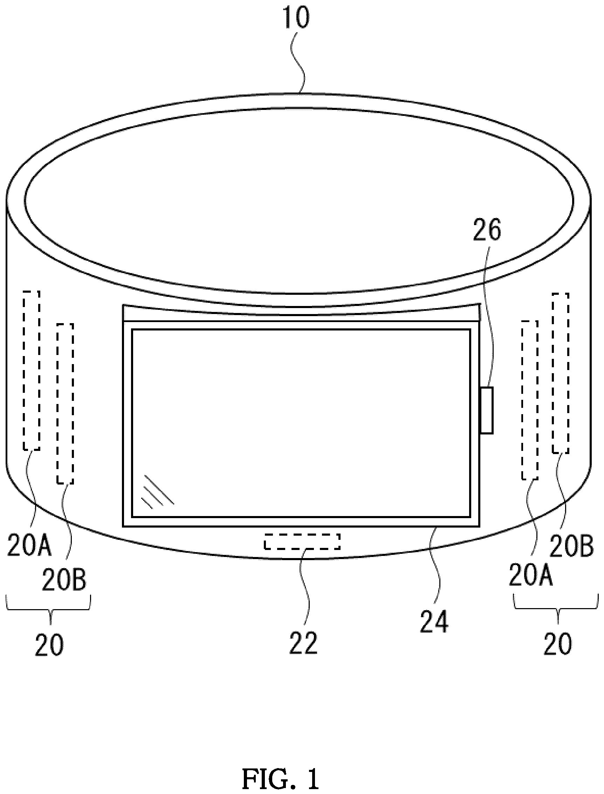

Figure 1 is a schematic configuration diagram of an analysis device 10 of the embodiment.
The analysis device 10 of the embodiment is made to be wearable on a person to be measured and is shaped like, for example, a band. The analysis device 10 is, for example, a wristband that is worn on a wrist, but is not limited to this and may be worn on an ancle. The analysis device 10 may also be shaped like, for example, a plane instead of a band, which may be sticked on the chest, the abdomen, or the like by means of an adhesive.
The analysis device 10 is provided with two or more electrode units 20 and a body temperature measuring unit 22 on the inside so that they are in contact with the arm of a person to be measured, and with a monitor 24 and a power switch 26 on the outside. Note that the analysis device 10 does not have to have the body temperature measuring unit 22. In such a mode, a body temperature acquisition unit 32 described later acquires the body temperature of a person to be measured from another device.
Each electrode unit 20 comprises a current electrode 20A and a voltage electrode 20B. The analysis device 10 applies electric current to the current electrodes 20A, and uses the voltage electrodes 20B to measure the potential difference occurring across the current path between the electrode units 20 being in contact with a person to be measured. Based on the potential difference, the analysis device 10 calculates the electric resistance of the person to be measured as bioimpedance (hereinafter referred to as "BI"), and based on the BI, determines an ingested component, which is a component ingested by the person to be measured.
While the current electrodes 20A and the voltage electrodes 20B are vertically long in the example in Figure 1, they are not limited to this and may have a horizontally long shape which lengthens in the circumferential direction of the analysis device 10.
The monitor 24 is, for example, a touch-panel display, through which a person to be measured performs an input operation on the analysis device 10 and a result of determining an ingested component is output. The power switch 26 accepts power-on and power-off operations of the analysis device 10.

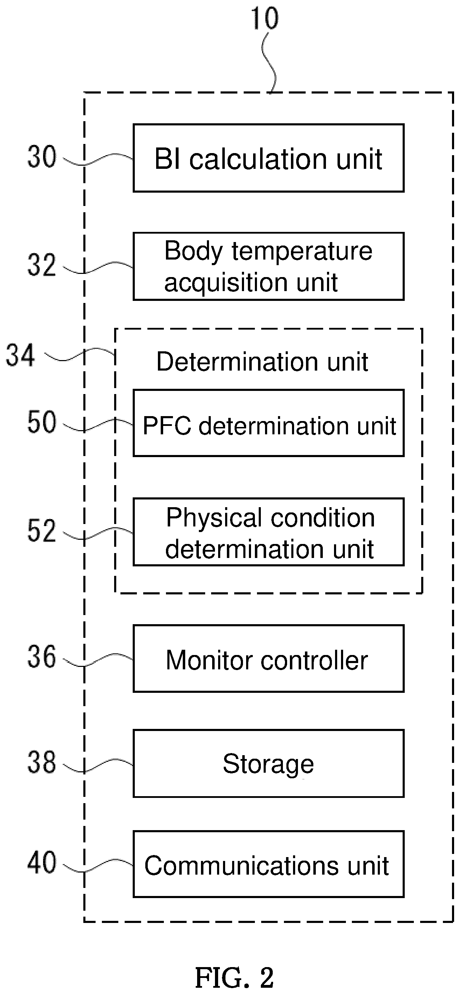

Figure 2 is a function block diagram of the analysis device 10 of the embodiment. The analysis device 10 comprises a BI calculation unit 30, the body temperature acquisition unit 32, a determination unit 34, a monitor controller 36, a storage 38, and a communications unit 40. Each function performed by the BI calculation unit 30, the body temperature acquisition unit 32, and the determination unit 34 shown in Figure 2 may, for example, be performed by an arithmetic unit provided on the analysis device 10 through activation of a program, or be realized by an ASIC (Application Specific Integrated Circuit) or other individual hardware components.

The BI calculation unit 30 calculates the BI of a person to be measured based on a measurement result obtained by using the electrode units 20 being in contact with the person to be measured (hereinafter referred to as "actual measured BI").

The body temperature acquisition unit 32 acquires the body temperature of a person to be measured from the body temperature measuring unit 22 as actual measured body temperature.

The determination unit 34 determines a component ingested by a person to be measured from diurnal and chronological changes in actual measured BI calculated by the BI calculation unit 30. Specifically, the determination unit 34 determines an ingested component, which is a component ingested by a person to be measured, based on a temporal change in actual measured BI and on reference change information. That is, as described later in detail, the determination unit 34 determines a component ingested by a person to be measured using a phenomenon of the BI of a person to be measured changing in accordance with a component ingested by the person to be measured.

The reference change information indicates a temporal change in BI according to a component ingested by a human, and is stored in the storage 42 in advance. As described later in detail, the reference change information of the embodiment includes reference PFC information, reference dehydration information, and reference excessive salt information.

The monitor controller 36 controls the monitor 24 so as to display a determination result obtained by the determination unit 34.

The storage 38 is, for example, a non-volatile memory, and contains various data, a program used for analysis processing, and the like. The various data includes the reference change information, a result of determination on an ingested component, and the like.

The communications unit 40 sends and receives data to and from a portable terminal device such as a smartphone owned by a person to be measured or another information processing device such as a server.

The determination unit 34 comprises a PFC determination unit 50 and a physical condition determination unit 52.

The PFC determination unit 50 uses the reference PFC information as the reference change information. The reference PFC information indicates a temporal change in BI for each ingested component resulting from a human ingesting protein (P), fat (F), and carbohydrate (C) which are ingested components. Protein, fat, and carbohydrate are also collectively referred to as nutrients in the description below.

Incidentally, diet-induced thermogenesis (DIT) is known to detect a change in body temperature before and after eating and thereby determine the degree of thermal productivity resulting from dietary intake, and digestion and absorption. Specifically, a higher-protein meal causes a larger increase in body temperature after eating, and a higher-carbohydrate and higher-fat meal causes a smaller increase in body temperature after eating.

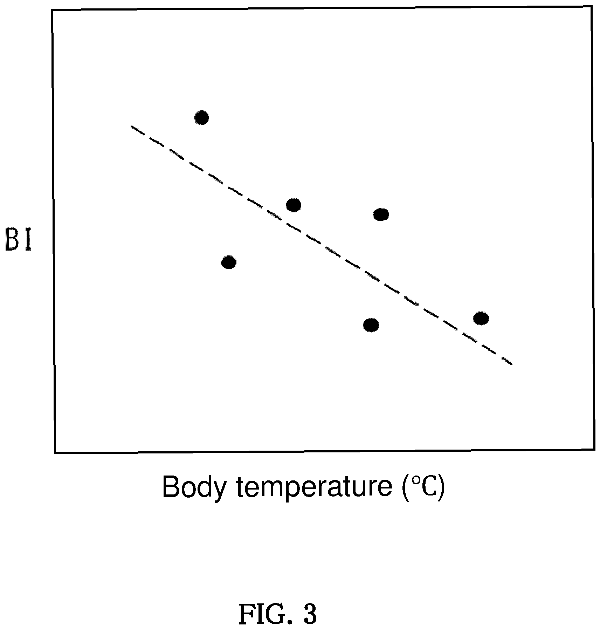

There is a correlation between such a change in body temperature after eating and a change in BI within a short period of time (a temporal change within dozens of minutes to a few hours), and BI falls as body temperature rises as shown in Figure 3. Note that the dashed line in Figure 3 is an approximate line. The PFC determination unit 50 therefore identifies a component ingested by a person to be measured from a temporal change in actual measured BI.

The PFC determination unit 50 determines that a component ingested by a person to be measured is protein, fat, or carbohydrate based on a temporal change in actual measured BI and on a temporal change for each nutrient indicated by the reference PFC information. This allows for identifying which of protein, fat, and carbohydrate the person to be measured has ingested as a nutrient.

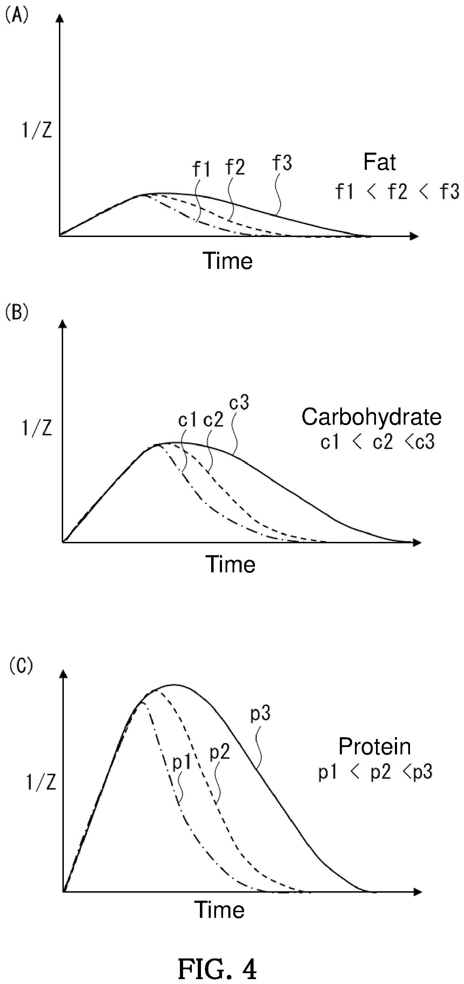

Figure 4 shows temporal changes of BI for nutrients ingested by a human. The vertical axis in Figure 4 is the reciprocal of bioimpedance Z (1/Z), where the bioimpedance Z is BI, and the horizontal axis is time. Figure 4 (A) shows temporal changes in BI resulting from the ingestion of fat, where the amounts of ingested fat increase in the order of the dash-dot line f1, the dashed line f2, and the solid line f3. Figure 4 (B) shows temporal changes in BI resulting from the ingestion of carbohydrate, where the amounts of ingested fat increase in the order of the dash-dot line c1, the dashed line c2, and the solid line c3. Figure 4 (C) shows temporal changes in BI resulting from the ingestion of protein, where the amounts of ingested fat increase in the order of the dash-dot line p1, the dashed line p2, and the solid line p3.

As shown in Figure 4, when a human ingests fat, carbohydrate, and protein, the peak height of 1/Z is the lowest for fat, and increases in the order of carbohydrate, and protein. Additionally, for all of fat, carbohydrate, and protein, the larger the amount of ingestion, the longer the time until 1/Z returns to the pre-ingestion level. Time until 1/Z returns to the level before the ingestion of a nutrient is referred to as the temporal change width in the description below.

As shown in Figure 4, the reference PFC information includes the maximum amount of change in BI (the maximum value of 1/Z) for each nutrient resulting from a human ingesting protein, fat, and carbohydrate. The PFC determination unit 50 of the embodiment compares the maximum amount of change in actual measured BI with the maximum amount of change for protein, fat, or carbohydrate indicated by the reference PFC information, and thereby determines that a nutrient ingested by a person to be measured is protein, fat, or carbohydrate.

As described above, the PFC determination unit 50 of the embodiment determines a nutrient ingested by a person to be measured based on 1/Z, where the maximum amount of change in BI for each nutrient is the maximum value of 1/Z, but this is not the only way, and the PFC determination unit 50 may determine a nutrient ingested by a person to be measured based on the minimum value of Z for each nutrient.

Additionally, the slope from the ingestion of fat, carbohydrate, and protein until 1/Z becomes the maximum varies as shown in Figure 4 (A) to (C). The PFC determination unit 50 may therefore determine a nutrient ingested by a person to be measured based on the slope of the temporal change in 1/Z or Z.

The reference PFC information further includes a relation between the amount of an ingested nutrient, which is protein, fat, or carbohydrate, and time until BI returns to the level before the ingestion of the nutrient (hereinafter referred to as the "temporal change width"). The temporal change width has different values according to the amount of ingestion of each nutrient as shown in Figure 4. That is, the larger the amount of an ingested nutrient, the larger the temporal change width.

The PFC determination unit 50 therefore determines the amount of a nutrient ingested by a person to be measured based on the temporal change width until actual measured BI returns to the level before the ingestion of the nutrient and on the first reference information. Specifically, the PFC determination unit 50 of the embodiment determines the temporal change width from when the actual measured BI starts changing until it returns to its original level. The PFC determination unit 50 then, after identifying a nutrient ingested by the person to be measured, compares the temporal change width for each amount of the ingested nutrient indicated by the reference PFC information with the temporal change width of actual measured BI to determine the amount of the ingested nutrient. Incidentally, the temporal change width for each amount of an ingested nutrient included in the reference PFC information and the temporal change width of actual measured BI are, for example, the full width at half maximum.

In this way, the PFC determination unit 50 can identify a nutrient ingested by a person to be measured from the peak height of actual measured BI, and can quantitatively determine the amount of a nutrient ingested by a person to be measured from the temporal change width of actual measured BI.

When the amount of an identified nutrient is a predetermined level or more, the PFC determination unit 50 may indicate to the person to be measured via the monitor 24 or the like that the nutrient has been ingested too much. The predetermined amount mentioned here may be, for example, a reference value determined in advance, or an average value over the past few days multiplied by a predetermined factor.

Note that the reference PFC information may include the maximum amount of change in BI and the temporal change width for each amount of ingestion for when protein, fat, and carbohydrate are mixed in predetermined proportions. For example, the reference PFC information may include the maximum amount of change in BI and the temporal change width for each amount of ingestion for when 50% protein and 50% fat are mixed and ingested, when 50% protein, 25% fat, and 25% carbohydrate are mixed and ingested, or the like. This allows for identifying a nutrient ingested by a person to be measured even when a plurality of nutrients are ingested at the same time.

While the reference PFC information described above is generated from, for example, actual measured values resulting from a plurality of adults ingesting nutrients, this is not the only way, and it may be generated from actual measured values of a person to be measured concerned. That is, the reference PFC information may be generated by acquiring a temporal change in BI within a predetermined period of time after a person to be measured ingests protein, fat, or carbohydrate. A component ingested by a person to be measured can be more accurately identified through generating in advance the reference PFC information according to the person to be measured.

When the reference PFC information is generated according to a person to be measured, the person to be measured is given a protein-prescribed diet (prescribed supplement) to ingest. The temporal change in BI is then recorded from the ingestion for a predetermined period of time, so that the reference change for 100% protein of the person to be measured is acquired. Likewise, the person to be measured is given a carbohydrate-prescribed diet and a fat-prescribed diet to ingest, so that the reference change for 100% carbohydrate and the reference change for 100% fat of the person to be measured are acquired, and these are set as the reference PFC information according to the person to be measured. A balanced mixed diet that is a mixture of 50% protein and 50% fat may also be given to a person to be measured to ingest as a prescribed diet to acquire the temporal change in BI.

The reference PFC information may include a temporal change in body temperature for each ingested component resulting from a human ingesting protein, fat, and carbohydrate. The reference PFC information in this mode includes, for example, the maximum amount of change and the temporal change width of body temperature elevation for when protein, fat, and carbohydrate are ingested. Note that the maximum amount of change in body temperature elevation increases in the order of carbohydrate, fat, and protein.

Given the above, the PFC determination unit 50 determines that a nutrient ingested by a person to be measured is one of protein, fat, and carbohydrate based on a combination of actual measured body temperature and actual measured BI and on the reference PFC information. For example, the PFC determination unit 50 compares an average value of the rate of change of actual measured body temperature and rate of change of actual measured BI (1/Z) with an average value of the rate of change of body temperature for each nutrient and rate of change of BI (1/Z) included in the reference PFC information, and identifies a nutrient ingested by the person to be measured. This allows for more accurately identifying a component ingested by a person to be measured. An average value of the temporal change width of actual measured body temperature and temporal change width of actual measured BI may also be used to identify the amount of a nutrient ingested by a person to be measured.

Incidentally, the determination of the PFC determination unit 50 with consideration for a temporal change in body temperature allows for separately determining a state of dehydration of a person to be measured and the ingestion of a nutrient. If a person to be measured is dehydrated, body temperature increases first and is followed by BI, but the ingestion of water causes body temperature and BI to decrease together, as described later. On the other hand, if body temperature decreases as BI increases, it can be determined that the person to be measured is not dehydrated and has ingested a nutrient. This allows for raising the accuracy of determining the ingestion of a component by a person to be measured.

The physical condition determination unit 52 determines a physical condition of a person to be measured based on a relation between a temporal change in actual measured body temperature acquired by the body temperature acquisition unit 32 and a temporal change in the electric resistance and on the reference change information. Note that the reference change information used by the physical condition determination unit 52 comprises the reference dehydration information and the reference excessive salt information.

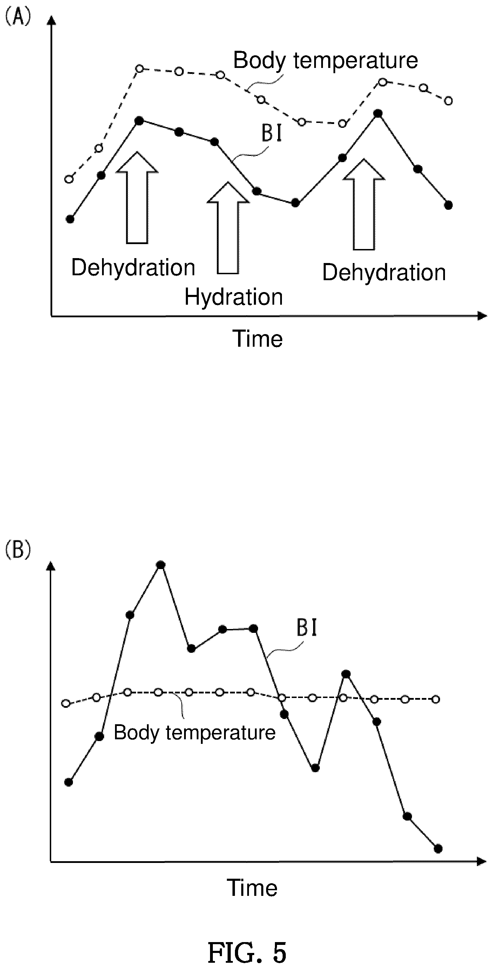

Figure 5 shows temporal changes of BI and body temperature according to physical conditions of a human. Figure 5 (A) is for a dehydrated human, and Figure 5 (B) is for a human with excessive salt.

As shown in Figure 5 (A), the reference dehydration information indicates that body temperature increases before BI increases and the ingestion of water causes body temperature and BI to decrease together. The physical condition determination unit 52 compares a combination of actual measured BI and actual measured body temperature with the reference dehydration information, and if they match each other, the physical condition determination unit 52 can determine that the person to be measured is dehydrated. In other words, if actual measured body temperature increases before actual measured BI increases and then actual measured body temperature decreases together with actual measured BI, the person to be measured is determined to be dehydrated.

As shown in Figure 5 (B), the reference excessive salt information indicates that there is no temporal change in body temperature while BI increases and then decreases. Incidentally, a human ingesting too much salt results in a phenomena where BI increases first due to absorption of salt (Na) and then decreases because a change in osmotic pressure causes water to be drawn in. Accordingly, excessive salt intake results in a temporal change in BI as shown in Figure 5 (B). Excessive salt, however, does not affect body temperature, and therefore body temperature remains constant. The physical condition determination unit 52 therefore compares a combination of actual measured BI and actual measured body temperature with the reference excessive salt information, and if they match each other, the physical condition determination unit 52 can identify the person to be measured as having edema due to excessive salt intake. In other words, if there is no temporal change in actual measured body temperature while actual measured BI increases and then decreases, the person to be measured is determined to have ingested too much salt.

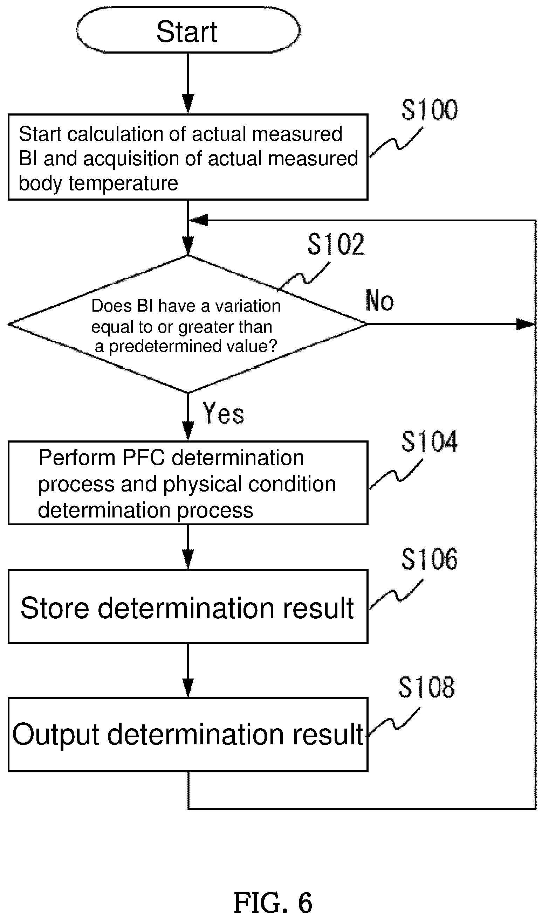

Figure 6 is a flowchart showing a flow of an analysis process performed by the analysis device 10. The analysis process is started when the power switch 26 is turned on after the analysis device 10 is worn by a person to be measured.

First, at a step S100, calculation of actual measured BI and acquisition of actual measured body temperature of the person to be measured are started.

At the next step S102, the determination unit 34 determines whether the actual measured BI has a variation equal to or greater than a predetermined value or not, and the process proceeds to a step S104 if the determination is affirmative. A variation equal to or greater than the predetermined value means a significant variation equal to or greater than a measurement error, and the predetermined value is determined in advance. If the determination is negative on the other hand, the calculation of actual measured BI and acquisition of actual measured body temperature of the person to be measured are repeatedly continued.

At the next step S104, based on the actual measured BI and the actual measured body temperature, the PFC determination unit 50 performs a PFC determination process to identify a nutrient ingested by the person to be measured, and the physical condition determination unit 52 performs a physical condition determination process to determine the physical condition of the person to be measured.

Note that the determination result to be obtained is one of an identification of a nutrient ingested by the person to be measured made by the PFC determination process, a determination that the person to be measured is dehydrated, and a determination that the person to be measured has ingested too much salt. That is, there is no such determination result that, for example, a nutrient ingested by a person to be measured is identified and at the same time the person to be measured is dehydrated.

Additionally, as described above, temporal changes in actual measured body temperature and actual measured BI approximately match each other when a person to be measured ingests a nutrient such as protein, fat, and carbohydrate. On the other hand, when a person to be measured is dehydrated or has ingested too much salt, temporal changes in actual measured body temperature and actual measured BI rarely match each other. Therefore, the PFC determination unit 50 may perform the PFC determination process when temporal changes in actual measured body temperature and actual measured BI of a person to be measured match each other, and the physical condition determination unit 52 may perform the physical condition determination process when temporal changes in actual measured body temperature and actual measured BI of a person to be measured do not match each other.

At the next step S106, the determination result obtained by the PFC determination process or the physical condition determination process is stored in the storage 38.

At the next step S108, the monitor controller 36 causes the monitor 24 to output (display) the determination result. The determination result may be output (sent) via the communications unit 40 to another information processing device.

Incidentally, the calculation of actual measured BI and the acquisition of actual measured body temperature are continued while the steps from S104 to S108 are being performed, and the analysis process is continued until the analysis device 10 is powered off.

(Second embodiment)

A second embodiment of the embodiment will be described below. Based on variations over one day or more in body composition of a person to be measured, the analysis device 10 of the embodiment corrects the amount of an ingested nutrient determined from diurnal and chronological changes in BI.

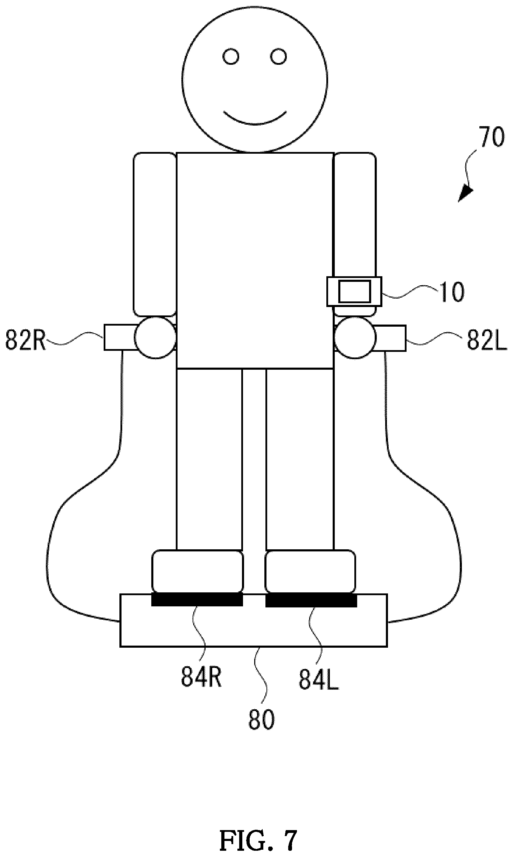

Figure 7 is a schematic configuration diagram of an analysis system 70 of the embodiment. The analysis system 70 comprises the analysis device 10 and a body composition analyzer 80. The body composition analyzer 80 shown in Figure 7 comprises electrode-equipped handgrips 82R and 82L, sole electrodes 84R and 84L, and a scale. The body composition analyzer 80 measures a difference in potential that occurs in a current path through the handgrips 82R and 82L and the sole electrodes 84R and 84L, thereby calculates the bioimpedance (BI) of the whole body and each body part of a person to be measured, and calculates a body composition value based on the BI and weight of the person to be measured. Note that the body composition value calculated by the body composition analyzer 80 is fat percentage, fat mass, fat-free mass, muscle mass, visceral fat mass, visceral fat level, visceral fat area, subcutaneous fat mass, basal metabolic expenditure, bone mass, body water percentage, BMI, intracellular fluid volume, extracellular fluid volume, or the like.

A person to be measured of the embodiment uses the body composition analyzer 80 to acquire the person's own body composition value one or more times per day. The body composition value acquired by the body composition analyzer 80 is sent to the analysis device 10 by means of a communications function.

Incidentally, the body composition analyzer 80 may be configured not to have the handgrips 82R and 82L but to comprise the sole electrodes 84R and 84L and the scale. The body composition analyzer 80 may also calculate a body composition value based on a difference in potential that occurs in a current path through the electrode units 20 provided on the analysis device 10 worn by a person to be measured and the electrodes provided on the body composition analyzer 80. A body composition value acquired by the body composition analyzer 80 may also be input to the analysis device 10 by a person to be measured in cases such as when the analysis device 10 and the body composition analyzer 80 cannot send and receive information through communication.

In addition, a person to be measured may wear two or more analysis devices 10 as the analysis system 70, and a body composition value may be calculated based on a difference in potential that occurs in a current path through the electrode units 20 that the two or more analysis devices 10 has and on a separately-measured weight of the person to be measured.

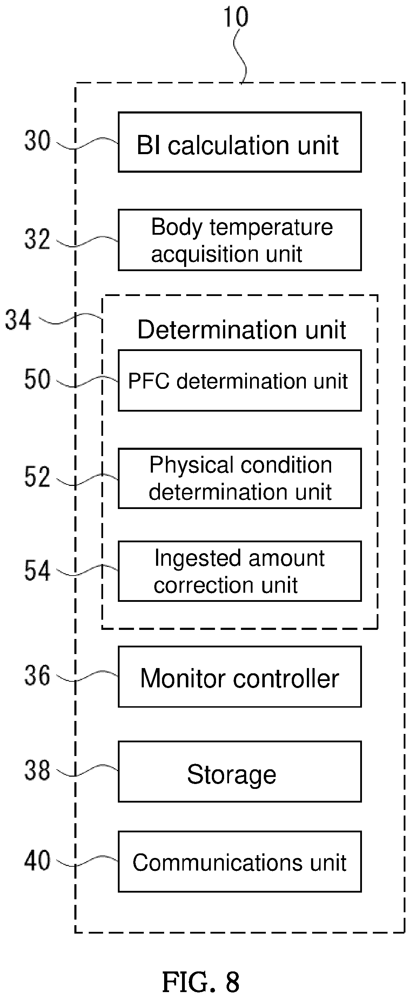

Figure 8 is a function block diagram relating to analysis functions that the analysis device 10 of the embodiment has. Note that the same components in Figure 8 as in Figure 2 are designated by the same symbols as in Figure 2, and their descriptions are omitted.

The determination unit 34 of the embodiment has an ingested amount correction unit 54. The ingested amount correction unit 54 corrects the amount of an ingested component (nutrient) determined by the PFC determination unit 50 based on variations over one day or more in body composition of the person to be measured (hereinafter referred to as the "ingested component correction function").

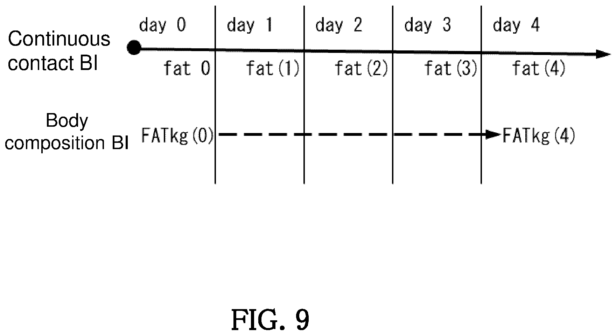

The ingested component correction function performed by the ingested amount correction unit 54 will be described with reference to a schematic diagram in Figure 9. Continuous contact BI in Figure 9 is the amount of fat ingested by a person to be measured determined from diurnal and chronological changes in BI obtained by the analysis device 10. Body composition BI is the amount of fat of a person to be measured calculated from BI obtained by the body composition analyzer 80. In other words, body composition BI is the daily amount of fat of a person to be measured.

The ingested amount correction unit 54 corrects the daily amount of fat ingested by a person to be measured using, for example, below-described Equation (1) and based on the amount of change in fat over days of the person to be measured. The amount of change in fat over days of a person to be measured, ΔFATkg, is shown for four days in Figure 9 and Equation (1), but this is just an example, and the amount of fat ingested by a person to be measured just needs to be corrected based on the amount of change in fat ΔFATkg over one day or more.

$$\Delta FATkg\ (FATkg(4) - FATkg(0)) = a*(fat(1) + fat(2) + fat(3) + fat(4)) \qquad (1)$$

As indicated by Equation (1), the amount of change in fat of a person to be measured, ΔFATkg, has a correlation with the amount of ingested fat, fat(n), summed over corresponding days. Therefore, the ingested amount correction unit 54 corrects the amount of ingested fat, fat(n), so that the amount of change in fat, ΔFATkg, matches the total of the amount of ingested fat, fat(n). Incidentally, the method of correction is, for example, to equally increase or decrease each of the daily amount of ingested fat, fat(n), so that the amount of change in fat, ΔFATkg, matches the total of the amount of ingested fat, fat(n).

Fat ingested by a person to be measured does not entirely and continually accumulate in the person to be measured. Ingested fat is consumed in daily activities of the person to be measured. Therefore, a term indicating the amount of daily activities of a person to be measured may be added to the right side of Equation (1). This takes the amount of fat consumed in activities into consideration for the amount of fat ingested by a person to be measured, and thus allows for correction with higher accuracy. Incidentally, for example, a value calculated by an activity monitor worn by a person to be measured is used as the amount of activity.

While Figure 9 and Equation (1) are for when the nutrient is fat, the ingested amount correction unit 54 may correct the amount of protein or carbohydrate ingested by a person to be measured using a body composition value that has a correlation with the amount of protein or carbohydrate ingested.

(Third embodiment)

A third embodiment of the embodiment will be described below. The analysis device 10 of the embodiment derives advice to maintain health for a person to be measured according to a determination result obtained by the determination unit 34.

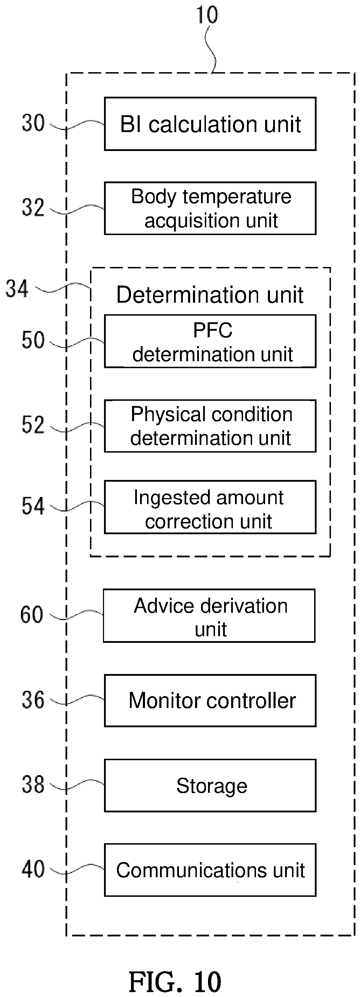

Figure 10 is a function block diagram relating to analysis functions that the analysis device 10 of the embodiment has. The analysis device 10 of the embodiment has an advice derivation unit 60.

The advice derivation unit 60 derives advice to maintain health for a person to be measured according to a determination result obtained by the determination unit 34. Advice to be derived is, for example, prepared in patterns according to determination results, and the patterns are stored in the storage 38. Derived advice is, for example, displayed on the monitor 24 or output as sound from a speaker (not shown), and is thus presented to the person to be measured.

Specific examples of the advice to be derived will be described next.

For example, if the physical condition determination unit 52 has determined excessive salt, the advice derivation unit 60 presents the person to be measured with advice to ingest potassium (K). This is aimed at promoting the excretion of sodium (Na) from the person to be measured before the person's blood pressure rises due to the excessive salt. Specific names of food including much potassium may also be presented. Such food items include, for example, spinach and lettuces as vegetables, bananas and apples as fruits, and avocados and wakame as other food items. Predetermined supplements, electrolyte drinks, or the like may also be presented as food items.

There are also cases where BI decreases significantly and the person to be measured continues to have a tendency for swelling even after a predetermined period of time has passed following a determination of excessive salt. In such cases, the advice derivation unit 60 may present advice to ingest a beverage with a diuretic effect. Beverages with diuretic effects include, for example, coffee and oolong tea. This is not the only way, and there may also be presentation of advice to promote excreting sodium along with sweat through exercise or bathing. This advice should also be presented with advice to hydrate.

For another example, if the PFC determination unit 50 results in a determination of excessive carbohydrate intake, the advice derivation unit 60 presents the person to be measured with advice to ingest high-fiber food and beverages in order to suppress the absorption of carbohydrate. High-fiber food includes, for example, vegetables, fruits, seaweed, and beans. High-fiber beverages include, for example, functional tea containing indigestible dextrin.

Moreover, the advice derivation unit 60 may also present a person to be measured with advice to do light exercise within a predetermined period of time (e.g., a few minutes to 60 minutes) from the ingestion of carbohydrate. This aims to cause skeletal muscles to take up sugar before elevation of blood sugar level or insulin secretion caused by absorbed carbohydrate and thereby prevent excessive insulin secretion due to a rapid increase in blood sugar level. Incidentally, specific examples such as rotating arms and legs, marching in place, and stretching may be presented as the light exercise.

Furthermore, the advice derivation unit 60 may also derive advice based on variations in BI, variations in weight, and variations in body composition over a few days, and the like. For example, the advice derivation unit 60 selects and presents a nutrient that should be ingested according to the physique or body composition of a person to be measured. Such nutrients that should be ingested include, for example, protein according to the muscle mass of the person to be measured, and fat-burning components according to the body fat ratio of the person to be measured. For another example, the advice derivation unit 60 may also present an exercise menu according to the physique or body composition of a person to be measured. Such exercise menus include, for example, an aerobic exercise menu according to the body fat ratio or visceral fat of the person to be measured, and a strength training menu according to a determination of the muscle mass score or SMI (skeletal muscle mass index) of the person to be measured.

(Fourth embodiment)

A fourth embodiment of the embodiment will be described below. While a component (nutrient, salt, or the like) ingested by a person to be measured is determined in the above-described embodiments, the analysis device 10 of the embodiment determines whether a person to be measured has ingested a predetermined specific component (hereinafter referred to as a "specified component") or not.

A specified component to be determined in the analysis device 10 of the embodiment is set by a person to be measured or the like. A specified component to be set is, for example, one of protein, fat, carbohydrate, and salt that can be determined. The determination unit 34 then determines whether the person to be measured has ingested the set specified component or not.

The determination unit 34 of the embodiment may also determine the status of injection of a set specified component (e.g., comparison with the previous day). It may also determine whether a person to be measured has excessively ingested a specified component concerned or not according to the status of ingestion. The determination of whether there has been an excessive ingestion or not is made by determining whether the amount of the ingested specified component is equal to or greater than a predetermined amount or not. The predetermined amount mentioned here may be, for example, a predetermined reference value or an average value over the past few days multiplied by a predetermined factor.

While the disclosure has been described with reference to the above embodiments, the technical scope of the disclosure is not limited to the scope provided by the embodiments. Various modifications or improvements can be made to the embodiments without departing from the gist of the disclosure, and those added with the modifications or improvements are also included in the technical scope of the disclosure.

While a description has been made for the above embodiments on a mode in which the analysis device 10 continually calculates the BI of a person to be measured after the power switch 26 is turned on, the disclosure is not limited to this. For example, the analysis device 10 may be provided with a meal start button. In this mode, a person to be measured wearing the analysis device 10 pushes the meal start button when starting a meal. This causes the analysis device 10 to measure the BI of the person to be measured for a predetermined period of time (e.g., an hour) after the push of the meal start button, and determine an ingested nutrient.

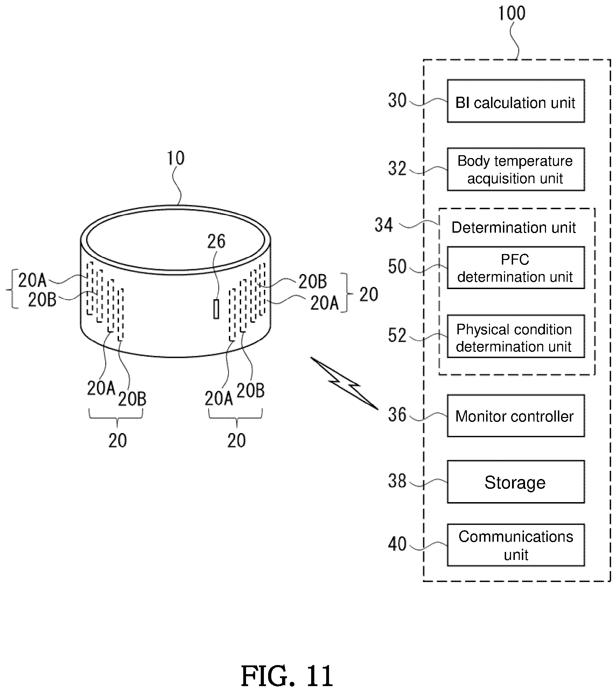

While a description has been made for the above embodiments on a mode in which the analysis device 10 determines a component ingested by a person to be measured, the disclosure is not limited to this. For example, as shown in Figure 11, an information processing device 100 such as a smartphone that a person to be measured has may have the BI calculation unit 30, the body temperature acquisition unit 32, the determination unit 34, the monitor controller 36, the storage 38, the communications unit 40, and the like. In this mode, the information processing device 100 receives actual measured body temperature and voltages detected by the voltage electrodes 20B from the analysis device 10, and determines a component ingested by the person to be measured. Incidentally, the analysis device 10 does not have to have the monitor 24 in this mode.

The information processing device 100 may also be a server, and in this mode, a result of determination of the server on a component ingested by a person to be measured is stored in the server. The person to be measured then causes a smartphone or the like to display the determination result stored in the server to confirm the determination result.

The analysis device 10 may also have three or more electrode units 20 as shown in Figure 11 (the analysis device 10 has four electrode units 20 in the example in Figure 11). In this mode, for example, when the physical condition determination unit 52 determines a change in salt and water of a person to be measured, it uses BI calculated from a difference in potential between electrode units 20 the distance between which is the shortest. This is because lymph and capillaries flowing closer to the surface of a human body are more susceptible to a change in salt.

On the other hand, when the PFC determination unit 50 determines a nutrient ingested by a person to be measure, it uses BI calculated from a difference in potential between electrode units 20 that are positioned farther apart than the electrode units 20 used by the physical condition determination unit 52. This is because a change in temperature caused by nutrient intake occurs more easily in deeper parts of a human body.

DESCRIPTION OF THE SYMBOLS

10: Analysis device
30: BI calculation unit (Calculation means)
32: Body temperature acquisition unit (Body temperature acquisition means)
34: Determination unit (Determination means)
54: Ingested amount correction unit (Correction means)
60: Advice derivation unit (Advice derivation means)

**Claims**

1. An analysis device comprising:

   calculation means for calculating electric resistance of a person to be measured based on a measurement result obtained by using a plurality of electrode units being in contact with the person to be measured; and
   determination means for determining a component ingested by the person to be measured based on reference information indicating a temporal change in the electric resistance according to a component ingested by a human and on a temporal change in the electric resistance calculated by the calculation means.

2. An analysis device comprising:

   calculation means for calculating electric resistance of a person to be measured based on a measurement result obtained by using a plurality of electrode units being in contact with the person to be measured; and
   determination means for determining whether the person to be measured has ingested a specified component or not based on reference information indicating a temporal change in the electric resistance according to a component ingested by a human and on a temporal change in the electric resistance calculated by the calculation means.

3. The analysis device according to claim 1 or 2,

   wherein the reference information includes a relation between an amount of the ingested component and time until the electric resistance returns to a level before ingestion of the component, and
   wherein the determination means determines an amount of the component ingested by the person to be measured based on time until the electric resistance calculated by the calculation means returns to a level before ingestion of the component and on the reference information.

4. The analysis device according to any one of claims 1 to 3, having
   correction means for correcting an amount of the component determined by the determination means based on variations over one day or more in body composition of the person to be measured.

5. The analysis device according to any one of claims 1 to 4,
   wherein the reference information is generated by acquiring a temporal change in the electric resistance within a predetermined period of time after the person to be measured ingests the component.

6. The analysis device according to any one of claims 1 to 5, having
   body temperature acquisition means for acquiring body temperature of the person to be measured,

   wherein the reference information includes a temporal change in the body temperature for each component resulting from a human ingesting the component, and
   wherein the determination means determines the component ingested by the person to be measured based on a combination of the body temperature acquired by the body temperature acquisition means and the electric resistance calculated by the calculation means and on the reference information.

7. The analysis device according to claim 6,
   wherein the reference information indicates that the body temperature increases before the electric resistance increases and that ingestion of water causes the body temperature to decrease along with the electric resistance.

8. The analysis device according to claim 6,
wherein the reference information indicates that there is no temporal change in the body temperature while the electric resistance increases and then decreases.

9. The analysis device according to any one of claims 1 to 8,
wherein the reference information indicates a temporal change in the electric resistance for each component resulting from a human ingesting the component, which is protein, fat, carbohydrate, or salt, or water while dehydrated.

10. The analysis device according to any one of claims 1 to 9, having
advice derivation means for deriving advice to maintain health for the person to be measured according to a determination result obtained by the determination means.

11. The analysis device according to any one of claims 1 to 10,
wherein the electrode units are made to be wearable on the person to be measured.

12. An analysis method having:

a first process for calculating electric resistance of a person to be measured based on a measurement result obtained by using a plurality of electrode units being in contact with the person to be measured; and
a second process for determining a component ingested by the person to be measured based on reference information indicating a temporal change in the electric resistance according to a component ingested by a human and on a temporal change in the electric resistance calculated by the first process.

13. An analysis program causing a computer to function as:

calculation means for calculating electric resistance of a person to be measured based on a measurement result obtained by using a plurality of electrode units being in contact with the person to be measured; and
determination means for determining a component ingested by the person to be measured based on reference information indicating a temporal change in the electric resistance according to a component ingested by a human and on a temporal change in the electric resistance calculated by the calculation means.

14. An analysis method having:

a first process for calculating electric resistance of a person to be measured based on a measurement result obtained by using a plurality of electrode units being in contact with the person to be measured; and
a second process for determining whether the person to be measured has ingested a specified component or not based on reference information indicating a temporal change in the electric resistance according to a component ingested by a human and on a temporal change in the electric resistance calculated by the first process.

15. An analysis program causing a computer to function as:

calculation means for calculating electric resistance of a person to be measured based on a measurement result obtained by using a plurality of electrode units being in contact with the person to be measured; and
determination means for determining whether the person to be measured has ingested a specified component or not based on reference information indicating a temporal change in the electric resistance according to a component ingested by a human and on a temporal change in the electric resistance calculated by the calculation means.

16. A computer-readable non-transitory storage medium holding the analysis program according to claim 13.

17. A computer-readable non-transitory storage medium holding the analysis program according to claim 15.

FIG. 1

10

| BI calculation unit |
| --- |

30

| Body temperature acquisition unit |
| --- |

32

Determination unit

34

| PFC determination unit |
| --- |

50

| Physical condition determination unit |
| --- |

52

| Monitor controller |
| --- |

36

| Storage |
| --- |

38

| Communications unit |
| --- |

40

FIG. 2

Body temperature (°C)

FIG. 3

FIG. 4

FIG. 5

Start

Start calculation of actual measured BI and acquisition of actual measured body temperature — S100

Does BI have a variation equal to or greater than a predetermined value? — S102

No

Yes

Perform PFC determination process and physical condition determination process — S104

Store determination result — S106

Output determination result — S108

FIG. 6

FIG. 7

10

```
┌ ─ ─ ─ ─ ─ ─ ─ ─ ─ ─ ─ ─ ─ ─ ─ ─ ─ ┐
```

30 — ┌─────────────────────────────┐
     │     BI calculation unit     │
     └─────────────────────────────┘

32 — ┌─────────────────────────────┐
     │    Body temperature         │
     │    acquisition unit         │
     └─────────────────────────────┘

34 — Determination unit

50 — ┌─────────────────────────────┐
     │   PFC determination unit    │
     └─────────────────────────────┘

52 — ┌─────────────────────────────┐
     │   Physical condition        │
     │   determination unit        │
     └─────────────────────────────┘

54 — ┌─────────────────────────────┐
     │   Ingested amount           │
     │   correction unit           │
     └─────────────────────────────┘

36 — ┌─────────────────────────────┐
     │     Monitor controller      │
     └─────────────────────────────┘

38 — ┌─────────────────────────────┐
     │         Storage             │
     └─────────────────────────────┘

40 — ┌─────────────────────────────┐
     │    Communications unit      │
     └─────────────────────────────┘

FIG. 8

FIG. 9

10

```
┌ ─ ─ ─ ─ ─ ─ ─ ─ ─ ─ ─ ─ ─ ─ ┐
```

30 —  BI calculation unit

32 —  Body temperature acquisition unit

34 —  Determination unit

50 —  PFC determination unit

52 —  Physical condition determination unit

54 —  Ingested amount correction unit

60 —  Advice derivation unit

36 —  Monitor controller

38 —  Storage

40 —  Communications unit

FIG. 10

100

30 — BI calculation unit

32 — Body temperature acquisition unit

34 — Determination unit

50 — PFC determination unit

52 — Physical condition determination unit

36 — Monitor controller

38 — Storage

40 — Communications unit

10

26

20A
20B
20B

20B
20A
20

20A 20B    20A 20B
20            20

# FIG. 11

## INTERNATIONAL SEARCH REPORT

| International application No. |
| --- |
| **PCT/JP2023/006774** |

### A. CLASSIFICATION OF SUBJECT MATTER

***A61B 5/0537***(2021.01)i
FI: A61B5/0537

According to International Patent Classification (IPC) or to both national classification and IPC

### B. FIELDS SEARCHED

Minimum documentation searched (classification system followed by classification symbols)

A61B5/0537

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); PubMed

### C. DOCUMENTS CONSIDERED TO BE RELEVANT

| Category* | Citation of document, with indication, where appropriate, of the relevant passages | Relevant to claim No. |
| --- | --- | --- |
| A | ANDROUTSOS, O. et al. Impact of eating and drinking on body composition measurements by bioelectrical impedance. Journal of human nutrition and dietetics: the official journal of the British Dietetic Association. April 2015, vol. 28, no. 2, pp. 165-171, <DOI:10.1111/jhn.12259><br>        tables 1-4 | 1-17 |
| A | DIXON, C. B. et al. The effect of a meal on measures of impedance and percent body fat estimated using contact-electrode bioelectrical impedance technology. European Journal of Clinical Nutrition. September 2013, vol. 67, no. 9, pp. 950-955, <DOI:10.1038/ejcn.2013.118><br>        tables 1, 2 | 1-17 |
| A | JP 2016-508755 A (ACCESS BUSINESS GROUP INTERNATIONAL LLC) 24 March 2016 (2016-03-24)<br>        abstract, paragraphs [0043], [0058]-[0072] | 1-17 |
| A | US 2020/0359937 A1 (PERFORMANCE ATHLYTICS) 19 November 2020 (2020-11-19)<br>        paragraphs [0032]-[0045] | 1-17 |

| ☐ Further documents are listed in the continuation of Box C. | ☑ See patent family annex. |
| --- | --- |

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **25 April 2023** | **16 May 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/006774**

| Patent document cited in search report | | | Publication date (day/month/year) | Patent family member(s) | | | Publication date (day/month/year) |
|---|---|---|---|---|---|---|---|
| JP | 2016-508755 | A | 24 March 2016 | US | 2014/0169400 | A1 | |
| | | | | abstract, paragraphs [0040], [0054]-[0068] | | | |
| | | | | WO | 2014/099240 | A1 | |
| | | | | KR | 10-2015-0096742 | A | |
| | | | | CN | 104994781 | A | |
| US | 2020/0359937 | A1 | 19 November 2020 | WO | 2019/046596 | A1 | |
| | | | | KR | 10-2020-0095457 | A | |
| | | | | CN | 111629661 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022026849 A
- JP 2000023936 A